# EUROPEAN PATENT APPLICATION

(11) **EP 1 820 528 A1**
(43) Date of publication of application: **22.08.2007**
(21) Application number: 06110137.4
(22) Date of filing: 20.02.2006
(51) Int. Cl.: A61M 16/08

(54) **Patient breathing circuit**

(71) Applicant: General Electric Company, Schenectady, NY 12345 (US)
(72) Inventor: Laurila, Santtu, Helsinki 00500 (FI); Ranta, Janne, Espoo 02710 (FI); Tamminen, Anne, Helsinki 00700 (FI); Holopainen, Timo, Helsinki 00510 (FI)
(74) Representative: Valkeiskangas, Tapio Lassi Paavali

(57) **Abstract**

The invention relates to patient breathing circuit for use in anaesthesia or respiratory care, the patient circuit comprising a gas source for creating gas mixture for patient breathing and circulating arrangement for delivering the gas mixture to the patient. The circulating arrangement comprising inspiration (9) and expiration tubes (8) through which gases flow to the patient and from the patient, the inspiration and expiration tubes (8,9) having machine ends and patient ends and the tubes (8,9) being provided with a Y-piece (12) at the patient end of the tubes enabling the flow of gases from the inspiration tube (9) to the patient (1) and from the patient (1) to the expiration tube (8). The Y-piece (12) is provided with a flow measuring sensor (2) having pressure lines (3) connected to the measuring device. The pressure lines (3) are arranged to run inside of an outer wall surface of the flow measuring sensor (2).

## Description

The invention relates to a patient breathing circuit for use in anaesthesia or respiratory care, the patient circuit comprising a gas source for creating gas mixture for patient breathing and circulating arrangement for delivering the gas mixture to the patient, the circulating arrangement comprising inspiration and expiration tubes through which gases flow to the patient and from the patient, the inspiration and expiration tubes having machine ends and patient ends, the tubes being provided with a Y-piece at the patient end of the tubes enabling the flow of gases from the inspiration tube to the patient and from the patient to the expiration tube, the Y-piece being provided with a flow measuring sensor having pressure lines connected to the measuring device.

Monitoring of ventilation is often difficult due to the limitations of available technology and monitoring equipment. Most devices utilized for measuring and monitoring ventilation are located distal to the patient, and therefore may not represent patient values. The best possible location for the measurement of patient values during anaesthesia and respiratory care should be as close to the patient as possible.

Ensuring adequate and optimum patient ventilation is the goal of every anaesthetist. Delivery of life support gases is based on pressure. However, without knowing the measured volume of exhalation, one cannot be sure that a breath occurred. The objective of ventilation is to use the least amount of pressure to generate the most appropriate volume for each breath. An excellent tool for helping to manage the patient's ventilation is patient spirometry that measures the contents of expired gases, airway pressure, flow and volume and computes compliance and resistance. In anaesthesia, patient spirometry enables both patient monitoring and anaesthesia circuit monitoring, which enables early detection of changes and helps to avoid ventilatory complications. In other words, it adds safety in ventilation. In critical care, patient spirometry is used to optimize the ventilator settings and to avoid complications. It also supports for treatment decisions and adds safety. It reduces risk of ventilator induced lung injury.

U.S. Patents No. 5,088,332 and 5,111,827 to Instrumentarium Corporation, describe a patient spirometry sensor, the D-lite flow sensor and gas sampler that allows the simultaneous measurement of airway gases, lung mechanics and metabolism. This patient spirometry measures standard gases (CO₂, O₂, N₂O, and anaesthetic agents) as well as airway pressures (peak, plateau, PEEP), lung volumes (minute and tidal), and graphically displays loops (pressure-volume and flow-volume) and curves (pressure and flow) breath-by-breath. Additional numeric displays include a calculation of dynamic compliance, airway resistance and trends. When the D-lite flow sensor is placed closed to the patient's airway, it provides continuous and accurate information on changes in the patient's ventilatory status.

The D-lite patient spirometry sensor described in the U.S. patents described above is attachable to and detachable from a patient breathing circuit Y-piece. The use of spirometry therefore generates more connections to a patient breathing circuit. Connections always create risks for leaks and endanger patient safety. The spirometry pressure tubes attached to the pressure ports and gas sampling tube attached to the sample port are currently situated outside the breathing circuit running from the patient end to the monitor. This "cable spaghetti" creates problems for both the hospital personnel and the patient safety.

The object of the invention is to obtain a patient breathing circuit by which the disadvantages of the prior art can be eliminated. This is achieved with the present invention. The patient breathing circuit of the invention is characterized in that the pressure lines are arranged to run inside of an outer wall surface of the flow measuring sensor.

An advantage of the invention over the prior art is that the amount of connections in breathing circuit is diminished decreasing the risk for leaks and hence ensuring better patient safety. Additionally, the space between the patient and the Y-piece becomes shorter since the spirometry sensor is integrated with the Y-piece diminishing the volume of dead space. By dead space is meant the quantity of exhaled gases, which return to the patient. The invention is also flexible, i.e. the invention can be used in connection with different constructions. In other words the invention can quite well be used in both coaxial and two limb patient breathing circuits. The expression Y-piece thus includes also connector for coaxially connecting inhalation and exhalation tubes.

The invention will now be described in greater detail with reference to the attached drawing in which
Figure 1 shows an example of the prior art spirometry sensor,
Figure 2 shows an example of the prior art breathing circuit system using the sensor shown on Figure 1,
Figure 3 shows one embodiment of the spirometry sensor arrangement used in the invention,
Figure 4 shows a machine end of the tube system shown in Figure 3,
Figure 5 shows the machine end connection of the system shown in Figures 3 and 5.
Figure 6 shows another embodiment of the spirometry sensor arrangement used in the invention,
Figure 7 shows a machine end of the tube system shown in Figure 6, and
Figure 8 shows the machine end connection of the system shown in Figures 6 and 7.

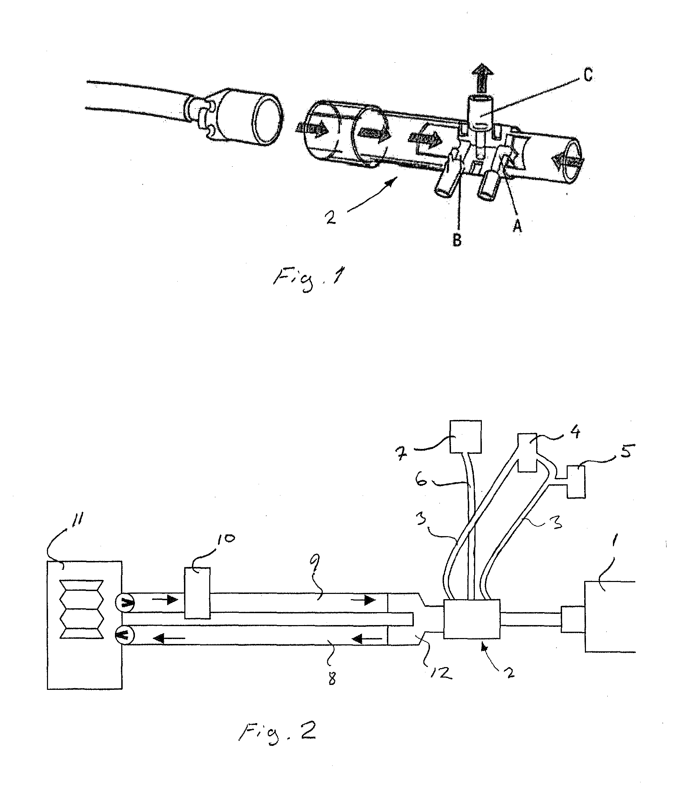

Figure 1 shows a prior art flow measuring and gas sampling detector, i.e. the spirometry sensor described in U.S. patents 5,088,332 and 5,111,827. This known sensor called the D-lite patient spirometry sensor is currently in use. A and B represent ports for pressure sensing tubes and C for gas analysis, as will be described below.

As told above the D-lite patient spirometry sensor in Figure 1 incorporates three ports: two for pressure sensing (A and B) and one for sidestream gas analysis (C). The velocity of gas flow is obtained when the dynamic pressure is measured by the two hollow tubes (A and B). On inspiration, gas moves for example from an anaesthesia machine to the patient (by point A), which measures the total pressure, and the same time the pressure at tube B is measured as the static pressure. The static pressure at B is subtracted from the total pressure at A to give the dynamic pressure. Dynamic pressure is proportional to the velocity of gas flow. D-lite sensor is designed to work in both directions; during expiration the process is reversed. Gas sample for CO₂, O₂, N₂O and anaesthetic agent measurement is taken through port C.

The structure described above must be seen only as an example, i.e. the structure can be modified in various ways. The structure can for example be materialized without gas sample possibilities etc.

Figure 2 shows a general view of the patient breathing circuit system with a current D-lite spirometry sensor. Reference number 1 shows a patient. Reference number 2 shows the spirometry sensor. Reference number 3 refers to pressure lines and reference number 4 to a pressure difference measuring device. Reference number 5 shows an absolute pressure measuring device. Reference number 6 shows a sampling line and reference number 7 a gas content measuring device. Reference number 8 shows an expiration tube and reference number 9 an inspiration tube. Reference number 10 refers to a CO2 absorber and reference number 11 shows a gas source, for example a respirator. Reference number 12 shows a Y-piece. The components and the system shown in Figure 2 are quite familiar to a person skilled in the art, and therefore the system and its components are not described here in detail.

The spirometry sensor 2 described in figure 1 is attachable to and detachable from a patient breathing circuit Y-piece 12, as presented in the Figure 2. The use of spirometry therefore generates more connections to a patient breathing circuit. As told before connections always create risks for leaks and endanger patient safety. The spirometry pressure lines 3 attached to ports A and B and gas sampling line 6 attached to port C are currently situated outside the breathing circuit creating "cable spaghetti". The high amount of separate cables, i.e. "cable spaghetti" in turn creates problems for both the hospital personnel and the patient safety.

In the embodiment of the present invention shown the flow measuring sensor 2 is integrated with the Y-piece 12 and the pressure lines 3 are arranged to run inside of an outer wall surface of the integrated flow measuring sensor 2 and the Y-piece 12. The flow measuring sensor can also comprise a gas sampling line 6 connected to a measuring device 7. In such an embodiment according to the invention the gas sampling line 6 is also arranged to run inside of the outer wall surface of the integrated flow measuring sensor 2 and the Y-piece 12. The term "integrated with" above means that the flow measuring sensor 2 and the Y-piece 12 are permanently connected to each other, i.e. the components 2 and 12 form a uniform structure. In other words the components 2 and 12 are fore example glued together or the components are fabricated as an integral unit.

In the embodiment of the invention described in Figures 3 - 5 the pressure lines 3 and the gas sampling line 6 are further arranged to run inside of the expiration 8 from the detector 2 to the machine end of the tube 8. In the embodiment shown in Figures 3 - 5 the lines 3, 6 are tubes that run freely in the gas flow channel of the expiration tube 8. It is however quite possible within the spirit of the invention to arrange the lines 3, 6 to run in the gas flow channel of the inspiration tube 9 as well. The embodiment shown in Figures 3 and 4 is a coaxially connecting structure. The invention is however not restricted to the structure shown but the invention can be used also in connection with two limb patient breathing circuits.

The structure shown in figures 3 and 4 forms a breathing circuit that is able to measure flow, pressure difference as well as content of the expired gas (CO₂, O₂, N₂O and anesthetic agents). This integration enables connection of a spirometry containing breathing circuit to a patient mask or intubation tube in much easier way without too many connections. As told before the amount of connections in breathing circuit is diminished decreasing the risk for leaks and hence ensuring better patient safety. Additionally, the space between the patient and the Y-piece becomes shorter since the spirometry sensor is integrated with the Y-piece diminishing the volume of dead space, i.e. the quantity of exhaled gases, which return to the patient.

The pressure and gas sampling tubes 3, 6 of the invention are running through the patient breathing circuit inside the expiratory tube 8, as shown in Figures 3 and 4. Lead-out of the tubes 3, 6 from the breathing circuit encounters at the machine end as shown in Figures 4 and 5. In the embodiment shown in the Figures the lead-outs of the tubes 3, 6 are arranged to the machine end connector piece 13 of the expiration tube 8. In the embodiment shown the lead-outs have been arranged so that the tubes 3, 6 run out essentially in a radial direction from the end connector piece 13. This is however not the only possibility but the lead-outs can also be arranged so that the tubes run out axially from the end connector piece 13. All the three tubes 3, 6 of the spirometry sensor are now in an orderly fashion at one place diminishing the number of single tubes near the patient. Expiratory tube 8 covers the pressure and gas sampling tubes 3, 6 of the sensor, which reduces the risk of tube fouling and sticking. Further, pressure and gas sampling tubes 3, 6 can now be made of lighter, less supportive material since the outer expiratory tube 8 covers them. In addition, sampling of gas is easier since the gas sampling tube 6 can advantageously be situated inside the expiratory tube 8, where the temperature is warmer diminishing condensation inside the tube.

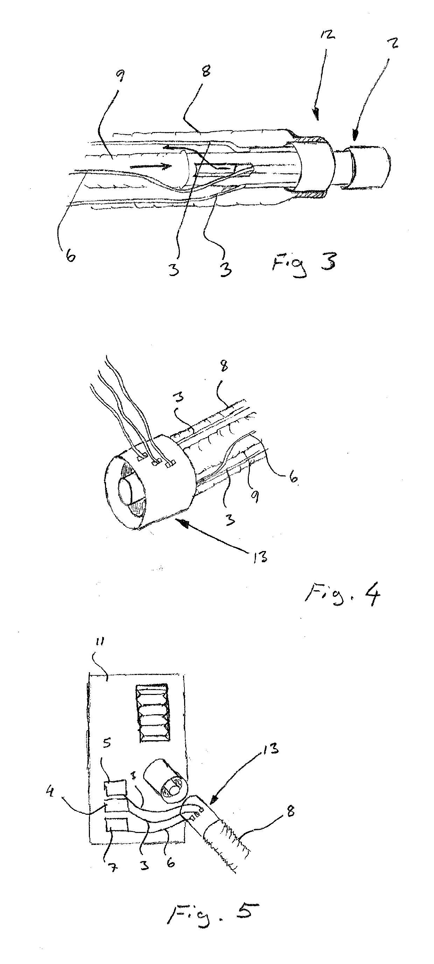

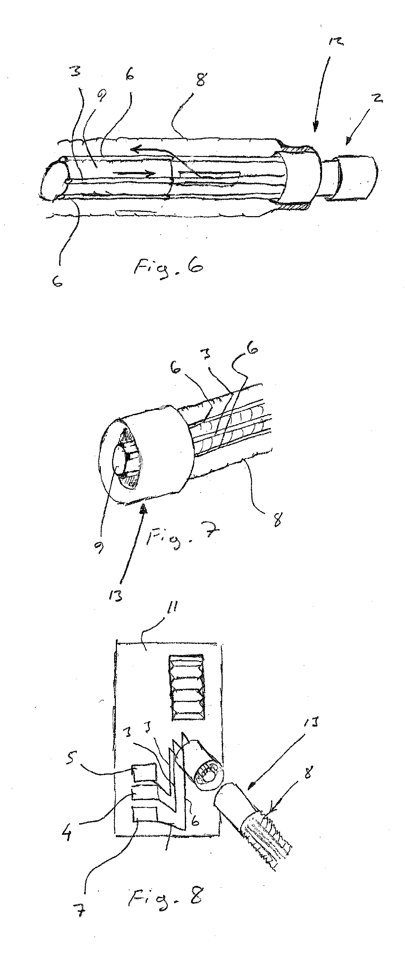

Figures 6 - 7 show another embodiment of the invention. The reference numbers used in Figures 6 - 7 refer to the details shown with corresponding reference numbers in Figures 3 - 5. In this embodiment shown in Figures 6 - 8 the lines 3,6 are lumina, i.e. cavities that are arranged to run in the outer wall material of the integrated flow measuring sensor 2 and the Y-piece 12 and also in the outer wall material of the expiration or inspiration tubes 8, 9. In other words in this embodiment pressure lines and gas sampling lines 3, 6 run in the wall separating expiration or inspiration gas flow from the surrounding atmosphere. The wall material separating expiration or inspiration gas flow has an inner wall surface and an outer wall surface. The inner wall surface defines the gas flow channel for expiration gas flow for example. In the embodiment shown in Figures 6 - 8 the lumina run in the material between the inner wall surface and the outer wall surface.

In the embodiment shown in Figures 6 - 8 the lead-outs can also be arranged so that the lines 3,6 run out axially from the end connector piece 13 as already referred above in connection with the embodiment of Figures 3 - 5.

The embodiments described above are by no means intended to restrict the invention, but the invention can be modified completely freely within the scope of the claims. The invention need not be materialized exactly in the way as described in the Figures but the entire structure or its details can be formed otherwise too.

## Claims

1. Patient breathing circuit for use in anaesthesia or respiratory care, the patient circuit comprising a gas source (11) for creating gas mixture for patient breathing and circulating arrangement for delivering the gas mixture to the patient (1), the circulating arrangement comprising inspiration (9) and expiration tubes (8) through which gases flow to the patient and from the patient, the inspiration and expiration tubes having machine ends and patient ends, the tubes (8,9) being provided with a Y-piece (12) at the patient end of the tubes enabling the flow of gases from the inspiration tube (9) to the patient (1) and from the patient (1) to the expiration tube (8), the Y-piece (12) being provided with a flow measuring sensor (2) having pressure lines (3) connected to the measuring device (4,5), **characterized in that that** the pressure lines (3) are arranged to run inside of an outer wall surface of the flow measuring sensor (2).

2. Patient breathing circuit according to claim 1, **characterized in that** the flow measuring sensor (2) further comprises a gas sampling line (6) connected to a measuring device (7), the gas sampling line being arranged to run inside of the outer wall surface of the flow measuring sensor (2).

3. Patient breathing circuit according to claim 1, **characterized in that** the flow measuring sensor (2) is integrated with the Y-piece (12) and that the pressure lines (3) are arranged to run also inside of the outer wall surface of the Y-piece.

4. Patient breathing circuit according to claim 3, **characterized in that** the flow measuring sensor (2) further comprises a gas sampling line (6) connected to a measuring device (7), the gas sampling line being arranged to run inside of the outer wall surface of the integrated flow measuring sensor (2) and the Y-piece (12).

5. Patient breathing circuit according to any of the claims 1 - 4, **characterized in that** the lines (3,6) are further arranged to run inside of an outer wall surface of the expiration or inspiration tube (8,9).

6. Patient breathing circuit according to any of the claims 1 - 5, **characterized in that** the lines (3,6) are tubes arranged to run in the gas flow channel of the integrated flow measuring sensor (2) and the Y-piece (12)/the expiration or the inspiration tubes (8,9).

7. Patient breathing circuit according to any of the claims 1 - 5, **characterized in that** the lines (3,6) are lumina arranged to run in the outer wall material of the integrated flow measuring sensor (2) and the Y-piece (12)/the expiration or inspiration tubes (8,9).

8. Patient breathing circuit according to claim 5, **characterized in that** the lines (3,6) are arranged to run inside of the outer wall surface of the expiration tube (8).

9. Patient breathing circuit according to claim 5, **characterized in that** lead-outs of the lines (3,6) are arranged to the machine end connector piece (13) of the expiration or inspiration tube (8,9).
